# EUROPEAN PATENT APPLICATION

(11) **EP 4 643 910 A1**
(43) Date of publication of application: **05.11.2025**
(21) Application number: 25172872.1
(22) Date of filing: 28.04.2025
(51) Int. Cl.: A61M 5/158, A61L 29/06, A61L 29/14

(54) **SELF-LUBRICATING REFLOWING INFUSION CATHETER**

(30) Priority: 01.05.2024 US 202463641341 P; 31.03.2025 US 202519096328
(71) Applicant: Medtronic MiniMed, Inc., Northridge, CA 91325-1219 (US)
(72) Inventor: Tong, Davy T., Northridge, 91325 (US); DANG, Kiem H., Northridge, 91325 (US); Chattaraj, Sarnath, Northridge, 91325 (US); Zhang, Guangping, Northridge, 91325 (US)
(74) Representative: Zimmermann & Partner Patentanwälte mbB

(57) **Abstract**

The present disclosure relates to a self-lubricating cannula for use in delivering a fluid medication to a subcutaneous site. The self-lubricating cannula comprises polyether block polyamide and an additive configured to facilitate the manufacture of a tipped self-lubricating cannula. The present disclosure also provides a method of making a self-lubricating cannula and a method of administering insulin using a self-lubricating cannula.

## Description

### CROSS-REFERENCE TO RELATED APPLICATIONS

This application claims the benefit of priority to U.S. Provisional Application No. 63/641,341, filed May 1, 2024, and US application no. 19/096,328 filed March 31, 2025, which is incorporated herein by reference in its entirety.

### TECHNICAL FIELD

The present technology relates generally to medical devices, and more particularly, to self-lubricating infusion cannulas for administering medications to individuals, such as insulin to diabetic patients.

### BACKGROUND

Millions of individuals having diabetes require insulin therapy to manage uncontrolled blood sugar levels (*i.e.,* blood glucose levels). As an alternative to multiple daily injections (syringe or pen injections), many people rely on small, wearable insulin infusion devices to manage their blood sugar. Typically, an infusion device includes a pump (which includes controls, a processing module, and batteries), a reservoir containing fluid medication (*e.g*. insulin), and an infusion set/sub-system. The infusion set/subsystem includes a cannula configured for subcutaneous insertion into the individual and a tubing system connecting the reservoir to the cannula. When the tubing in the infusion sub-system is minimal and the pump is adhered to skin, the pump system is wearable and called a patch pump. The cannula and tubing are part of the infusion sub-system in a patch pump. An infusion device where the pump is not worn against the skin but is tethered by a longer tubing is called a tethered pump, and the cannula and tubing are referred to as an infusion set. In either the patch pump or the tethered pump, the cannula is inserted subcutaneously and maintained at the infusion site for multiple days to enable delivery of the fluid medication. Cannulas provide a passageway for delivering the medication to the individual subcutaneously.

The manufacturing process of cannulas involves modification of the distal end of the cannula such that it is "tipped", or "tip-formed", to facilitate penetration of the tissue of an individual. The process of tipping involves modifying the distal end of the cannula in order to reduce the force required for penetration into and minimize injury to the tissues of the body. In the tipping process, a cannula is pressed into a heated mold, and takes on the shape and features of the mold. For instance, the features of the heated mold provide for cannula tip that narrows and may have rounded edges. The cannula tip geometry is optimized to reduce penetration force and discomfort in a patient during insertion into the body. Tipping is common for cannulas that are configured to contain a needle on the inside of its lumen.

The most popular and widely used cannulas are made of polytetrafluoroethylene (PTFE) and fluoroethylenepropylene (FEP). These biocompatible polymers are lubricious and allow for ease of manufacturing, including the formation of the tip geometry. Cannulas made from these polymers can reliably deliver insulin to an individual for multiple days before performance degrades and the cannula must be removed and replaced.

However, one drawback of using PTFE and FEP polymers is that they are composed of per- and polyfluroalkyl substances (PFAS), long lasting chemicals which have been associated with adverse effects to human health and are difficult to break down. Since the PFAS do not degrade easily, they can potentially build up over time and accumulate in the body and environment. Studies have revealed a correlation between exposure to certain PFAS and adverse effects on reproductive ability, developmental delays in children, an increased risk of cancers, reduction of the body's immune system to fight infections, interference with the body's natural hormones, and an increase in cholesterol levels and the risk of obesity.

There is a demand for biocompatible cannulas comprised of PFAS-free materials that are suitable for the tipping manufacturing process and can deliver insulin to the tissue of an individual over several days while maintaining good performance. Such a cannula should be made of a material that reliably facilitates the tipping process. A PFAS-free cannula would not only benefit millions of diabetic patients who need subcutaneous infusions of insulin, but any individual that needs subcutaneous infusions of a fluid medication.

### SUMMARY

This disclosure provides for a PFAS-free, self-lubricating cannula configured for subcutaneous insertion and exhibits desirable properties, such as reflow and self-lubrication. The self-lubricating cannula described herein comprises a tip formed by a process involving a heated mold and resists adhering to the heated mold. In addition, methods of making and using the self-lubricating cannula, including administering insulin to diabetic individuals, are disclosed herein.

In one aspect, the disclosure provides for a self-lubricating cannula comprising a polyether block polyamide and an additive, wherein the self-lubricating cannula is configured for delivering insulin to an individual in need thereof and the self-lubricating cannula resists sticking to a heated mold. Also provided is a self-lubricating cannula for use in delivering insulin to an individual in need thereof.

In one aspect, the disclosure provides a method of making a self-lubricating cannula, comprising the steps of: combining a polyether block polyamide and an additive to form a mixture, extruding the mixture to form a cannula, cutting the cannula to a desired length, and treating the distal end of the cannula with a heated mold to form a tip; wherein the self-lubricating cannula is for use in delivering insulin to an individual in need thereof, and the self-lubricating cannula resists sticking to a heated mold. In another aspect, a self-lubricating cannula formed by any of the methods described herein is provided.

In another aspect, the disclosure provides a method of administering insulin to an individual in need thereof, comprising: providing a self-lubricating cannula provided herein; inserting the self-lubricating cannula into the individual; and delivering insulin via the self-lubricating cannula into the individual in need thereof.

In yet another aspect, the disclosure provides an infusion device comprising: a housing configured to be positioned at the skin of a patient at an infusion site; a reservoir configured to store a fluid medication, the reservoir configured to be received by the housing; and a cannula configured for subcutaneous insertion into a tissue of the patient at the infusion site, wherein the cannula is configured to be self-lubricating and comprises a polyether block polyamide and an additive.

Various embodiments disclosed herein are provided as examples and do not limit the subject technology.

### BRIEF DESCRIPTION OF THE DRAWINGS

FIG. 1 summarizes certain general aspects of an exemplary cannula shown as flared onto a bushing or needle guide.
FIG. 2 illustrates an example of the overall cannula and cannula tip manufacturing process.
FIG. 3 is a photo of an exemplary cannula with a needle inside the tipped cannula.
FIGS. 4 shows photos of prepared cannulas after tip-forming comprising polyether block polyamide without siloxane (FIG. 4A) and with 1.5% siloxane (FIG. 4B.)
FIG. 5 is a drawing of a cannula example.

### DETAILED DESCRIPTION

### I. Cannula Overview

The cannula is a crucial component of infusion therapy and serves as the interface between the infusion device and the patient's subcutaneous tissue. A cannula provides the ability to convey fluid medication, such as insulin, to a targeted location on an individual's body (*i.e.* the infusion site) over a period of days (*e.g.* 1-2 days, 2-3 days, up to 6-7 days). Some cannulas are for subcutaneous insertion and can be 22-30 gauge. Some cannulas are made of a synthetic polymer and may also be known as a plastic catheter.

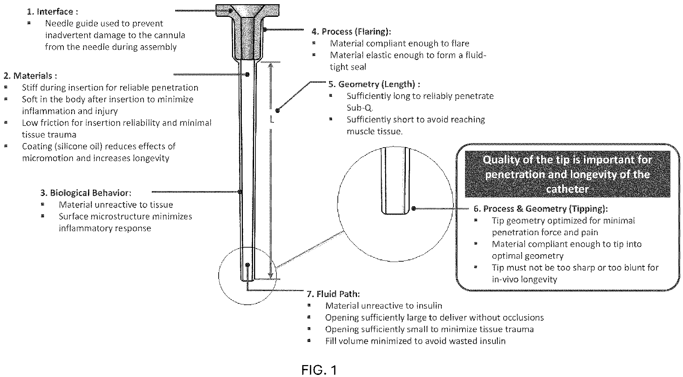

FIG. 1 is an illustration of a cannula mated to a bushing, which can function as a needle guide. The bushing connects the cannula to the rest of the infusion device. The bushing and cannula together function to provide a sealed fluid delivery path for a drug (*e.g*. insulin) into subcutaneous tissue of a patient (*e.g.* diabetic patients).

### A. Foreign Body Response and Cannula-Related Complications

The implantation of a cannula in human tissue triggers an immunological response known as the foreign-body response. This response manifests as an acute inflammatory reaction localized at the insertion site and may encompass the epidermis, dermis, and subcutaneous adipose tissue. Beyond the immunological response, the insertion process itself can induce mechanical trauma. This trauma can affect cells and connective tissue along the cannula's path, potentially damaging basement membranes, the extracellular matrix, and structural proteins. Disruption of the vascular network, including lymphatic vessels, arterioles, capillaries, and venuoles, can further compromise the tissue microenvironment and lead to fluid accumulation and potential clotting.

Another complication associated with cannula implantation is infusion site-loss or site-reduction. This phenomenon is believed to be partially mediated by encapsulation of the cannula by fibrous tissue. This and the quality of infused insulin, which may be affected by temperature and the interaction of insulin and materials it contacts (*e.g.* reservoir and fluid path during storage, filling, and delivery.) The encapsulation process can lead to inconsistent and unreliable medication delivery. In the case of inconsistent insulin, an individual is likely to experience blood glucose variability. The exact mechanisms underlying site-loss/reduction remain unclear, but likely involve a complex interplay between factors like localized inflammation, intraluminal coagulation within the cannula, and progressive fibrotic tissue proliferation. In addition, the movement of the cannula during daily activities can exacerbate the host response and contribute to ongoing tissue irritation.

The immunological response, tissue trauma, and infusion site-loss described above results in the failure of the cannula, the loss of effective insulin delivery, and ultimately uncontrolled blood glucose levels. In order to effectively deliver insulin and control blood glucose levels, the infusion set is replaced which involves removing failed cannula and inserting a fresh cannula, often on a different infusion site. One way to prolong the longevity of a cannula is to reduce or minimize the foreign body response, mechanical trauma, and infusion site-loss by optimizing the cannula tip.

### B. Cannula Features

The material of the self-lubricating cannula is stiff to facilitate penetration and insertion into patient tissue, and is resistant to bending or kinking, which could block the flow of insulin. The self-lubricating cannula material is compliant enough to flare and conform to a bushing or needle guide, yet elastic enough to form a fluid-tight seal around the bushing or needle guide. The self-lubricating cannula material is unreactive to patient tissue.

The *in vivo* performance of the self-lubricating cannula is impacted by its surface microstructure. A smooth surface reduces protein adherence to the cannula, potential occlusions, and minimizes the inflammatory response. The smooth surface also reduces friction during insertion, which helps reduce insertion force and tissue trauma. A smoother cannula surface can prolong the consistent delivery of insulin and avoid infusion site-loss for a longer period of time compared to a rougher surface. The smoothness of a surface may be evaluated by visually examining the surface with the aid of an instrument such as a microscope or SEM.

The quality of the self-lubricating cannula tip is critical for penetration and longevity of the cannula *in vivo.* The tip geometry of the cannula is optimized for minimal penetration force and causing minimal pain to the patient. To increase longevity of the cannula *in vivo,* the tip needs to strike a balance between being sharp and blunt. A sharp tip reduces the force needed to pierce the tissue, minimizing discomfort during insertion. After the cannula is inserted into the tissue, a degree of bluntness in the tip would help reduce tissue damage during any subsequent movement of the cannula tip within the body. Reducing the effects of micromotion of the cannula in the body by including an optional coating of silicone oil may increase cannula longevity.

The opening of the self-lubricating cannula is sufficiently large to deliver a fluid medication (*e.g*. insulin) without forming occlusions, and sufficiently narrow to minimize trauma to patient tissue during insertion and placement at the infusion site. In addition, the fill volume of the cannula is minimized to reduce the amount of insulin that is wasted after disposing the used cannula.

### C. General Manufacturing Process

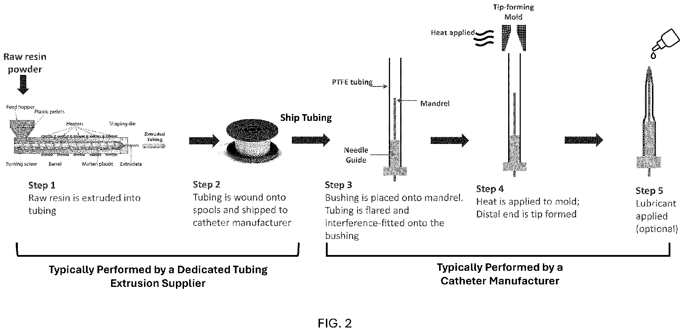

An overview of conventional cannula manufacturing and tip-formation is shown in FIG. 2. The conventional cannula fabrication process begins with adding the raw materials (*i.e.* a premixed copolymer/additive mixture) into a feed hopper of a screw extruder machine. The screw is rotated at a predetermined speed and temperature controllers connected to heating/cooling elements on the barrel to maintain the temperature at the set-point temperatures. The extrudate leaves the die, it can either be set to the desired shape or its shape can be altered and then set to shape, and the resulting catheter is cooled. The term "catheter" may be interchangeable with "cannula" as used herein. The catheter can be extruded on conventional manufacturing equipment and specific processing parameters related to the particular resin(s) may be obtained from the supplier. The extruded catheter is then cut to length and flared onto a mandrel mounted bushing by interference fitting, which is also known as friction fit or pressed fit. The distal end of the catheter is then tip-formed using a heated and lubricated mold. The tip-forming process requires a heated mold that is typically lubricated to prevent the catheter from sticking. Finally, the tipped cannula may be treated with a lubricant.

In the manufacturing process described above, polyether block polyamide resin and an additive are combined and thoroughly premixed before being added to the feed hopper of the screw extruder. The integration of the additive throughout the cannula material is important for the self-lubrication and reflow properties exhibited by the cannula.

The most crucial and difficult process step is the tip-forming process. A tip-forming mold determines the geometry of the distal portion of the cannula, features such as a rounded tip or tapered shape. The mold is designed to provide an optimized tipped catheter, where the shape and dimensions facilitate insertion into the body and reduce tissue trauma. The tip-forming process requires pre-heating a tip-forming mold to a predetermined temperature in order to reflow the resin of the tubing and force the tip of the tubing to conform to the shape of the mold. A silicone/siloxane lubricant is applied to the mold surfaces just prior to tip-forming. The distal end of the cannula (also known as a catheter tubing) is forced and pressed into the mold. A mandrel is used to hold, guide, and center the cannula with the mold during tip-forming. The distal end is held in the mold for a predetermined dwell time to enable the distal end of the cannula in contact with the mold to reflow and conform to the dimensions of the mold. After the predetermined dwell time is completed, the cannula is retracted from the mold. A successfully formed tip will have a smooth surface while also conforming to the dimensions of the molding surfaces.

### D. PFAS-Free Cannula Alternatives

Polytetrafluoroethylene (PTFE) and fluorinated ethylene propylene (FEP) have been the top materials of choice for cannula formation. PTFE can be reflowed when heated and is more lubricious than other resins, thus conforming to the shape of the mold and reducing the chance of sticking to the molding surfaces. Despite the lubricity of PTFE, mold surfaces are typically lubricated during fabrication in order to facilitate tip-forming and reduce sticking of the catheter to the mold.

PTFE and FEP are among the hundreds of chemicals categorized as perfluoroalkyl and polyfluoroalkyl substances (PFAS). PFAS are characterized by a chain of carbon atoms bonded to fluorine atoms and have been referred to as "forever chemicals" because of their persistence and resistance to degradation. There is a growing awareness of PFAS accumulating in the soils, water, and living organisms. The extent of the adverse impact of forever chemicals on human health and the environment is the subject of ongoing study. In the meantime, this disclosure provides for an alternative to the PTFE and FEP cannulas that are currently marketed. In some embodiments, the self-lubricating cannula described herein is PFAS-free.

A suitable alternative to PFAS-free materials is not obvious and there are many manufacturing and mechanical reasons why a material may not be appropriate for use in an infusion cannula. Hence the prevalence of PTFE and FEP cannula and catheters. Unlike PTFE and FEP, many other resins either do not reflow well or become extremely "sticky" when heated and adhere to molding surfaces. Some materials are inferior because they do not possess sufficient stiffness and will result in the cannula kinking or buckling during insertion, thus damaging the cannula. Some materials may not be sufficiently soft, which can lead to tissue trauma and inflammation due to the cannula moving while implanted in the body. Other resins might not be biocompatible and may react with the patient's tissue.

Materials, such as polyether block polyamide, also known under the tradename Pebax^{®} (Arkema), have been excluded from cannula manufacturing, despite being biocompatible and having demonstrated *in vivo* performance because of poor reflow and stickiness when heated, which fouls the tip-forming process. Suitable cannulas configured for insulin infusion devices composed of polyether block polyamide are unavailable because their manufacturing is not considered feasible.

This disclosure provides for cannulas comprising a polyether block polyamide and an additive, wherein the cannulas exhibit self-lubricating and reflow properties. With the inclusion of the additive, resins that were previously not considered as suitable materials for cannula production are now available for use in the preparation of a cannula as described herein.

The term "self-lubricating" refers to the ability of the cannula to provide for a lubricious, or slippery character inherent to the cannula material. The self-lubricating character permits the cannula to be formed with a heated mold without sticking to the mold upon release from the mold surface. The self-lubricating character of the self-lubricating cannulas disclosed herein is attributed to the inherent property of the cannula material, which is a combination of the polyether block polyamide and additive, rather than a separate coating of a lubricant to the surface of the cannula.

In some embodiments, the tip-forming process does not require lubrication of the heated mold or to the surface of the untipped canula, thus reducing the number of steps and lubrication material needed in the tip-forming process.

The term "reflow" refers to the process where the cannula material melts during the tip-forming process. When treated with a heated mold, the cannula melts, conforms to the dimensions of the mold, and then solidifies when cooled.

The combination of the polyether block polyamide and additive as disclosed herein provides self-lubricating and reflow properties that facilitate the tip-forming process. After a predetermined dwell time in the mold, the tipped cannula may be removed from the mold without adhering or sticking to the mold surface, thus preserving the integrity of the newly formed tip. The result is a self-lubricating cannula with regular and smooth surface tips. Without the inclusion of the additive, the tip forming process results in the cannula sticking to the mold and a poorly formed tip. The resulting tip has, for instance, irregular edges, rough surfaces, or a form that deviates from the mold dimensions.

In some embodiments, wherein after contact of the self-lubricating cannula with a heated mold, the surface of the self-lubricating cannula having contact with the heated mold is smoother compared to the surface of an analogous additive-free cannula having contact with a heated mold.

In some embodiments, wherein after contact of the self-lubricating cannula with a lubricated heated mold, the surface of the self-lubricating cannula having contact with the heated mold is smoother compared to the surface of an analogous additive-free cannula having contact with the lubricated heated mold.

### E. Polyether Block Polyamide

The self-lubricating cannula disclosed herein comprises a polyether block polyamide and an additive. Polyether block polyamide is a block copolymer made up of rigid polyamide blocks and soft polyether blocks. Polyether block polyamide is also known as PEBA, poly(ether-block-amide), polyether polyamide block copolymer, polyether polyamide copolymer, polyamide block polyether, polyamide polyether block copolymer, polyamide polyether copolymer. The term "copolymer" refers to any polymer formed from two or more monomers. Polyether block polyamide is a thermoplastic elastomer and an example of a copolymer.

Polyether block polyamide is obtained by the polycondensation of a carboxylic acid polyamide with an alcohol termination polyether. The general chemical structure of polyether block polyamide is: 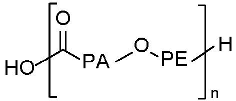 The repeat unit of the polyamide component (PA) of the block copolymer may have varying carbon chain lengths, for instance 6 carbons (PA6, Nylon 6), 11 carbons (Nylon 11), or 12 carbons (PA12, Nylon 12). For example: 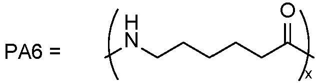 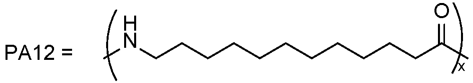 The polyether block polyamide may be substituted, for instance, N-substituted with methyl or acetyl. The polyether component (PE) of the block copolymer may have varying monomeric carbon chain lengths, for instance 2 carbons or 4 carbons, and examples include polytetramethylene ether glycol (PTMG), and poly(ethyleneoxide) (PEO), as shown below: 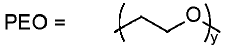 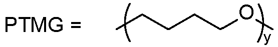 The terms "n", "x", and "y", refer to an integer and indicate the number of repeat units.

The raw material used in the preparation of the self-lubricating cannula is a polyether block polyamide resin. Polyether block polyamide is available from the manufacturer Arkema under the tradename of PEBAX^{®} and from Evonik Industries under the tradename VESTAMID^{®} E. The PEBAX^{®} resin is plasticizer free. A list of eight PEBAX^{®} block copolymers are listed in Table 1 along with their physical properties.

**Table 1.**

| **Character** | **Conditions** | **Standard** | **UNITS** | **7233** | **7033** | **6333** | **5533** | **4533** | **4033** | **3533** | **2533** |
|---|---|---|---|---|---|---|---|---|---|---|---|
| Shore Hardness | Instantaneous | ISO 868 | Shore D | 69 | 69 | 64 | 54 | 46 | 42 | 33 | 27 |
| | | | Shore A | - | - | - | - | 92 | 90 | 82 | 77 |
| | After 15 Seconds | ISO 868 | Shore D | 61 | 61 | 58 | 50 | 41 | 35 | 25 | 22 |
| | | | Shore A | - | - | - | - | 90 | 89 | 80 | 74 |
| Density | | ISO 1183 | g/cm³ | 1.01 | 1.01 | 1.01 | 1.01 | 1.01 | 1.00 | 1.00 | 1.00 |
| Melting Point | | ISO 11357-1/-3 | °C | 174 | 172 | 169 | 159 | 147 | 160 | 144 | 134 |
| Vicat Softening Temp. | Method A 10 N | ISO 306 | °C | 164 | 164 | 157 | 142 | 111 | 131 | 77 | 58 |
| Humidity Absorption | 23°C, 50% RH | ISO 62 | % | 0.7 | 0.7 | 0.7 | 0.6 | 0.4 | 0.5 | 0.4 | 0.4 |
| Water Absorption | 23°C, 24h in Water | ISO 62 | % | 0.9 | 0.9 | 1.1 | 1.2 | 1.2 | 1.2 | 1.2 | 1.2 |
| Melt Index | 235°C / 1kg | ISO 1133 | g/10 mn | 4 | 6 | 5 | 7 | 9 | 9 | 8 | 10 |
| CLTE - Linear Coeffcient | From -40°C to 140°C | ISO 11359-1/-2 | 10⁻⁵/°C | 12 | 16 | 14 | 17 | 20 | 20 | 21 16 | 10 |
| Surface Resistivity | | IEC 62631-3-2 | Ohm/sq | 2E+15 | 8E+13 | 3E+13 | 1E+13 | 4E+12 | 8E+12 | 2E+13 | 1E+13 |
| Volumic Resistivity | | IEC 62631-3-1 | Ohm. cm | 4E+13 | 6E+13 | 9E+12 | 2E+12 | 6E+11 | 2E+12 | 4E+11 | 3E+11 |
| Flexural Modulus | | ISO 178 | MPa | 513 | 390 | 285 | 170 | 86 | 77 | 21 | 12 |
| Tensile Modulus | | ISO 527 - 1/-2 | MPa | 542 | 384 | 240 | 161 | 81 | 71 | 18 | 10 |
| Tensile - Stress At Yield | v= 50 mm/min | ASTM D 638 type IV | MPa | 26 | 22 | 18 | 12 | - | - | - | - |
| Tensile - Strain At Yield | | | % | 18 | 20 | 22 | 25 | - | - | - | - |
| Tensile - Stress At Break | | | MPa | 56 | 54 | 53 | 52 | 42 | 40 | 39 | 32 |
| Tensile - Strain At Break | | | % | >300 | >350 | >350 | >450 | >550 | >450 | >600 | >750 |
| Impact Strength* (IZOD) At + 23°C | Unnotched | ISO 180 | J/m | N | N | N | N | N | N | N | N |
| | Notched | | | 192 | N | N | N | N | N | N | N |
| Impact Strength* (IZOD) At - 40°C | Unnotched | | | N | N | N | N | N | N | N | N |
| | Notched | | | 50 | 50 | 110 | N | N | N | N | N |
| Impact Strength* (Charpy) At + 23°C | Unnotched | ISO 179 | kJ/m2 | N | N | N | N | N | N | N | N |
| | Notched | | | 15^{C} | 120^{P} | N | N | N | N | N | N |
| Impact Strength* (Charpy) At - 30°C | Unnotched | | | N | N | N | N | N | N | N | N |
| | Notched | | | 10^{C} | 10^{C} | 20^{C} | N | N | N | N | N |

| | | | | | | | | | | | |
|---|---|---|---|---|---|---|---|---|---|---|---|
| N: No break C: Complete break P: Partial break | | | | | | | | | | | |

A polyether block polyamide, such as PEBAX^{®}, has measurable physical properties such as shore hardness, density, melting point, humidity, absorption, water absorption, flexural modulus, and tensile modulus, etc. as listed in Table 1. These properties may be measured by standardized tests, for example, those developed by ASTM International, ISO, and IEC.

In some embodiments, the polyether block polyamide is PEBAX^{®}, which may be obtained from a commercial supplier such as Arkema as a resin. In some embodiments, the polyether block polyamide is selected from the group consisting of PEBAX^{®} 2533, PEBAX^{®} 3533, PEBAX^{®} 4033, PEBAX^{®} 4533, PEBAX^{®} 5533, PEBAX^{®} 6333, PEBAX^{®} 7033, and PEBAX^{®} 7233, or a combination thereof. In some embodiments, the polyether block polyamide is selected from the group consisting of PEBAX^{®} 3533, PEBAX^{®} 5533, and PEBAX^{®} 7233.

In some embodiments, the polyether block polyamide disclosed herein has a physical property profile comprising two or more physical properties listed in Table 1, *e.g.* shore hardness, density, melting point, humidity, absorption, water absorption, flexural modulus, and tensile modulus, wherein the physical property profile is equivalent to the physical property profile of an PEBAX^{®} polyether block polyamide described in Table 1. In some embodiments, the polyether block polyamide disclosed herein has a physical property profile comprising two or more physical properties listed in Table 1, *e.g.* shore hardness, density, melting point, humidity, absorption, water absorption, flexural modulus, and tensile modulus, wherein the physical property profile is equivalent to the physical property profile of PEBAX^{®} 3533, PEBAX^{®} 5533, or PEBAX^{®} 7233, as described in Table 1. In some of the foregoing embodiments, the physical property profile is composed of three or more, four or more, five or more, six or more, or seven or more of the aforementioned physical properties.

In some embodiments the polyether block polyamide has a physical property profile equivalent to the physical property profile of an PEBAX^{®} polyether block polyamide selected from the group consisting of PEBAX^{®} 2533, PEBAX^{®} 3533, PEBAX^{®} 4033, PEBAX^{®} 4533, PEBAX^{®} 5533, PEBAX^{®} 6333, PEBAX^{®} 7033, and PEBAX^{®} 7233, wherein the PEBAX^{®} property profile is listed in Table 1. In some embodiments, the polyether block polyamide disclosed herein may have physical property profile equivalent to the physical property profile of PEBAX^{®} 3533, PEBAX^{®} 5533, and PEBAX^{®} 7233, wherein the PEBAX^{®} property profile is listed in Table 1.

"Equivalent", as used herein, is plus or minus 0.1% to 20%, plus or minus 0.1% to 10%, plus or minus 0.1% to 5%, or plus or minus 0.1% to 2% of the values listed in Table 1. In some embodiments the term "equivalent" is replaced by "about."

### F. ADDITIVE

The self-lubricating cannula described herein comprises an additive. The additive provides the self-lubricating character of cannula. In one aspect, the additive is introduced early in the cannula manufacturing process, where it is combined with the polyether block polyamide resin to form a paste. Thus, the additive and polyether block polyamide are intimately combined and mixed. The resulting catheter, which is later subjected to the tip-forming process, comprises the additive incorporated throughout the self-lubricating cannula, which is an important feature because it provides the reflow and non-sticking qualities desired during the tip forming process. Simply lubricating the surfaces of a heated mold or an untipped cannula with siloxane and then pressing the cannula into the mold results in a poorly formed tip if the additive is not incorporated with the polyether block polyamide into the cannula material. In one embodiment, the self-lubricating cannula comprises a homogeneous mixture of polyether block polyamide and additive.

An example of an additive is siloxane, or silicone, which is a class of chemical compounds having alternating silicon (Si) and oxygen (O) atoms. An example of a siloxane chemical formula is -(R₂SiO)ₙ-, where n is the number of repeating units, and R is an organic substituent such as methyl, ethyl, propyl, phenyl, etc., or R is an aminoalkyl, hydroxy, hydrogen, or vinyl group, for example. Siloxanes may be linear, cyclic, branched, or crosslinked. Siloxanes include oligomers and polymeric structures (polysiloxanes).

The macromolecular backbone of polysiloxanes is made up of a Si-O-Si units. The Si atoms are substituted with organic groups, such as C1-C10 hydrocarbon groups. An example of polysiloxane is polydimethylsiloxane (PDMS), where two methyl groups are bonded to each Si atom.

High molecular weight polysiloxane refers to a polysiloxane having a weight average molecular weight (Mw) of at least 5000 Da, at least 10,000 Da, at least 30,000 Da, at least 50,000 Da, at least 100,000 Da, at least 500,000 Da, at least 1,000,000 Da, or at least 2,000,000 Da. In some embodiments, high molecular weight polysiloxane is a polysiloxane having a Mw of at least 50,000 Da. In some embodiments, high molecular weight polysiloxane is a polysiloxane having a Mw of at least 100,000 Da. In some embodiments, high molecular weight polysiloxane is a polysiloxane having a Mw of at least 500,000 Da. In some embodiments, high molecular weight polysiloxane is a polysiloxane having a Mw of at least 1,000,000 Da. The Mw can be determined from molecular weight distribution data obtained from analysis by gel permeation chromatography. In some embodiments, the Mw of the polysiloxane is similar to the polysiloxane Multibase^{™} MB50-017 Masterbatch.

Polysiloxane is available from the manufacturer Dupont under the tradename Multibase^{™}. One example of a polysiloxane is Dow Corning MB50-017 Masterbatch (equivalent to rebranded Multibase^{™} MB50-017 Masterbatch), which is a pelletized formulation of an ultra-high molecular weight siloxane polymer dispersed in thermoplastic polyurethane carrier. The siloxane content of Dow Corning MB50-017 is 50%.

Other Multibase^{™} polysiloxanes include MB50-001, MB50-001 G2, HMB-0221, HMB-6301, MB25-501, MB50-002, MB25-035, MB25-502, AMB-12235, MB25-235, MB50-313, MB50-321, MB50-801, MB50-802, MB50-314, MB50-320, MB50-004, MB50-007, MB50-008, MB40-006, HMB-1103, MB50-011, MB50-320, HMB-1103, MB50-010, MB50-012, MB50-315, and HMB-1903.

The additive may be dispersed in a polymer carrier to facilitate its incorporation with polyether block polyamide during manufacturing. Examples of polymer carriers include thermoplastic polyurethane, polypropylene homopolymer (PPH), low density polyethylene (LDPE), linear low-density polyethylene (LLDPE), high density polyethylene (HDPE), ethylene-vinyl acetate (EVA), high impact polystyrene (HIPS), acrylonitrile butadiene styrene (ABS), styrene acrylonitrile (SAN), polyoxymethylene (POM), ethylene methyl acrylate copolymer (EMA), Nylon 6 (PA6), copolyester ether (COPE), polyethylene terephthalate (PET), and polycarbonate (PC), or a combination thereof.

In some embodiments, the additive promotes reflow of the self-lubricating cannula and reduces the adhesion of the self-lubricating cannula surface to a heated mold. In some embodiments, the additive is a siloxane. In some embodiments, the siloxane is polysiloxane. In some embodiments the polysiloxane is a high molecular weight siloxane polymer. In some embodiments, the additive is an alkylated polysiloxane. In some embodiments, the additive is polydimethylsiloxane. In some embodiments, the additive is Multibase^{™} MB50-017 Masterbatch. In some embodiments, the additive is dispersed in a polymeric carrier, such as thermoplastic urethane.

The amount of the additive in the self-lubricating cannulas disclosed herein is sufficient to provide a self-lubricating quality (*e.g*. non-stick to the heated mold, promoting smooth, regular surfaces) and retains the other desirable properties of the cannula, such as rigidity. It is also desirable to minimize the concentration of additive to avoid any possible leaching from the self-lubricating cannula into the fluid path, or to avoid any possible reaction with an individual's tissue.

In some embodiments, the self-lubricating cannula comprises: less than than 10 wt%, less than 7 wt%, less than 5 wt%, less than 3 wt%, less than 2 wt %, less than 1.5 wt%, or less than 1 wt% of the additive, and at least 0.1 wt% of the additive.

In some embodiments, the self-lubricating cannula comprises: 0 .1 wt% to 10 wt%, 0.1 wt% to 7 wt%, 0.1 wt% to 5 wt%, 0.1 wt% to 3 wt%, 0.1 wt% to 2 wt%, 0.1 wt% to 1.5 wt%, 0.1 wt% to 1 wt%, 0.5 wt% to 10 wt%, 0.5 wt% to 7 wt%, 0.5 wt% to 5 wt%, 0.5 wt% to 3 wt%, 0.5 wt% to 2 wt%, 0.5 wt% to 1.5 wt%, 0.5 wt% to 1 wt%, 0.7 wt% to 10 wt%, 0.7 wt% to 7 wt%, 0.7 wt% to 5 wt%, 0.7 wt% to 3 wt%, 0.7 wt% to 2 wt%, 0.7 wt% to 1.5 wt%, 0.7 wt% to 1 wt%, 1 wt% to 10 wt%, 1 wt% to 7 wt%, 1 wt% to 5 wt%, 1 wt% to 3 wt%, 1 wt% to 2 wt%, or 1 wt% to 1.5 wt% of the additive.

In some embodiments, the self-lubricating cannula comprises: about 10 wt%, about 9.5 wt%, about 9 wt%, about 8.5 wt%, about 8 wt%, about 7.5 wt%, about 7 wt%, about 6.5 wt%, about 6 wt%, about 5.5 wt%, about 5 wt%, about 4.5 wt%, about 4 wt%, about 3 .5 wt%, about 3 wt%, about 2.5 wt%, about 2 wt%, about 1.5 wt%, about 1 wt%, or about 0.5 wt % of the additive.

### G. Self-lubricating Cannulas Configured with Needles

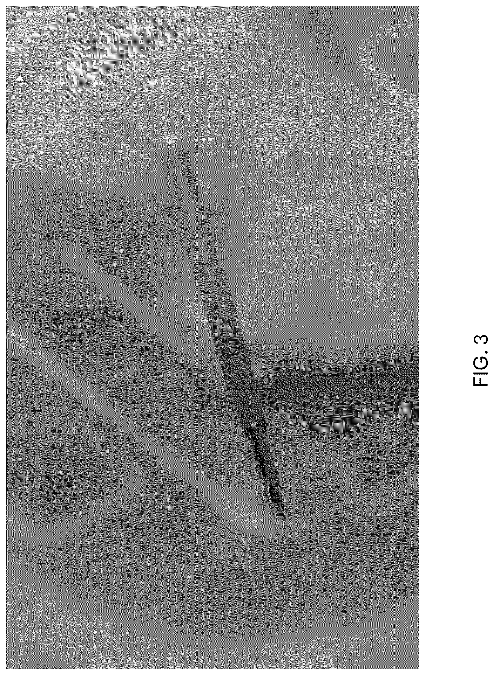

A needle may optionally be used to facilitate insertion of a cannula by puncturing the tissue (e.g. skin) of an individual in order to enable a cannula to be inserted and positioned at the intended site. A cannula can be configured to surround the outer surfaces of a needle. For instance, FIG. 3 shows a photo of a needle inside the lumen of a tipped cannula. Alternatively, the needle can be configured to surround the outer surfaces of the cannula.

In some embodiments, the self-lubricating cannula further comprises a needle located within the self-lubricating cannula. In some embodiments, the self-lubricating cannula further comprises a needle located on the exterior of the self-lubricating cannula. In some embodiments, the self-lubricating cannula is configured for subcutaneous insertion into a tissue of a diabetic patient. In some embodiments, the cannula is part of an infusion set/sub-system.

In some embodiments, the self-lubricating cannula comprises a polyether block polyamide and a siloxane, wherein: the self-lubricating cannula is configured for delivering insulin to an individual in need thereof; the self-lubricating cannula resists sticking to a heated mold; the siloxane is a high molecular weight polysiloxane (such as Multibase^{™} MB50-017); the polyether block polyamide is PEBAX^{®} (such as PEBAX^{®} 3533, PEBAX^{®} 5533, and PEBAX^{®} 7233); and the amount of siloxane is 0.1 wt% to 5 wt% (such as about 2 wt%, 1.5 wt%, and about 1 wt%).

In some embodiments, the self-lubricating cannula comprises a polyether block polyamide and a siloxane, wherein: the self-lubricating cannula is configured for delivering insulin to an individual in need thereof; the self-lubricating cannula resists sticking to a heated mold; the siloxane is a high molecular weight polysiloxane (such as Multibase^{™} MB50-017); the polyether block polyamide has a physical property profile comprising three or more physical properties selected from the group consisting of shore hardness, density, melting point, humidity, absorption, water absorption, flexural modulus, and tensile modulus; the physical property profile is equivalent to the physical property profile of an PEBAX^{®} polyether block polyamide described in Table 1 (*e.g.* PEBAX^{®} 3533, PEBAX^{®} 5533, and PEBAX^{®} 7233); and the amount of siloxane is 0.1 wt% to 5 wt% (such as about 2 wt%, 1.5 wt%, and about 1 wt%).

In some embodiments, the self-lubricating cannula comprises a polyether block polyamide and a siloxane, wherein: the self-lubricating cannula is configured for delivering insulin to an individual in need thereof; the self-lubricating cannula resists sticking to a heated mold; the siloxane is a high molecular weight polysiloxane (such as Multibase^{™} MB50-017), wherein the Mw of the polysiloxane is at least 10,000 Da, 50,000 Da, 100,000 Da, or 500,000 Da; the polyether block polyamide has a physical property profile comprising three or more physical properties selected from the group consisting of shore hardness, density, melting point, humidity, absorption, water absorption, flexural modulus, and tensile modulus; the physical property profile is equivalent to the physical property profile of an PEBAX^{®} polyether block polyamide described in Table 1 (*e.g.* PEBAX^{®} 3533, PEBAX^{®} 5533, and PEBAX^{®} 7233); and the amount of siloxane is 0.1 wt% to 5 wt% (such as about 2 wt%, 1.5 wt%, and about 1 wt%).

In some embodiments, the polyether block polyamide and siloxane is a homogeneous mixture.

### II. Methods of Using a Self-Lubricating Cannula to Deliver Fluid Medications

The disclosure provides for a method of using a self-lubricating cannula described herein to administer a fluid medication to an individual in need thereof. The individual in need thereof refers to a subject in need of a subcutaneous infusion of a fluid medication (*e.g.* insulin), and includes humans, pigs, dogs.

The fluid medication can be any medication where subcutaneous delivery is desired. Examples of subcutaneous medications include insulin, growth hormone, epinephrine, opioids, heparin, and allergy medications. Medications that require prolonged delivery to an individual over a period of days can benefit from use of the self-lubricating cannula.

Administration of a fluid medication may be carried out with an infusion device that contains the cannula. The infusion device includes a pump (which includes controls, a processing module, and batteries), a reservoir containing fluid medication (*e.g.* insulin), and an infusion set/subsystem, which includes the cannula described herein for subcutaneous insertion into the individual and a tubing system connecting the reservoir to the cannula. An infusion set refers to both an external infusion set connecting a tethered pump through a long tubing to a cannula, as well as an internal infusion set such as within a body worn patch pump (*i.e.,* the pump, cannula and tubing are all within the patch device).

In some embodiments, the disclosure provides a method of administering insulin to an individual in need thereof, comprising: providing a self-lubricating cannula as described herein; inserting the self-lubricating cannula into the individual; and delivering insulin via the self-lubricating cannula into the individual in need thereof.

The self-lubricating cannula is inserted subcutaneously at an infusion site located on the abdomen, thighs, hips, upper arms, lower back, or buttocks of the individual in need thereof. During insertion into the individual in need thereof, the self-lubricating cannula resists bunching or kinking. Insertion results in the cannula being implanted in the individual. The individual can continue to "wear" the self-lubricating cannula during this period. The term "to wear" as used herein, refers to having the cannula implanted in the individual after insertion.

Once implanted, the self-lubricating cannula is in contact with *(i.e.* implanted in) the individual in need thereof for at least 3, at least 4, at least 5, at least 6, at least 7, at least 8, at least 9, or at least 10 continuous days before the cannula is replaced or removed. The implantation of the self-lubricating cannula is maintained as long as the flow of the fluid medication (*e.g.* insulin) is sufficient to benefit the individual being administered the medication. In an individual with diabetes, the beneficial administration of insulin results in the effective control of blood glucose levels. When foreign body responses, inflammatory reactions, clotting, or any other adverse reaction occurs to cause the infusion site to fail, the blood glucose level will increase despite administration of larger doses of insulin. This is a signal that the individual no longer benefits from the infusion therapy and the cannula should be removed with a fresh cannula inserted at a different infusion site. Currently, manufacturers recommend removing the cannula and inserting it at a different site on the body every 3 days, and some cannulas up to 7 days. Exceeding this time increases the chance for tissue damage, infection, scarring, and increase in blood glucose.

In some embodiments the individual in need thereof is a human. In some embodiments, the individual is a diabetic human. In some embodiments, the individual is a diabetic swine.

In some embodiments, the distal end of the self-lubricating cannula comprises a tip configured to penetrate the skin of the individual in need thereof. In some embodiments, the self-lubricating cannula is configured to be worn (*i.e.* implanted) for at least 2 days, at least 3 days, or at least 3 days at a single site on a diabetic patient. In some embodiments, the self-lubricating cannula is in contact with the individual in need thereof for at least 3, at least 4, at least 5, at least 6, at least 7, at least 8, at least 9, or at least 10 continuous days before the self-lubricating cannula is replaced or removed.

In some embodiments, the self-lubricating cannula is used to deliver a fluid medication to an individual in need thereof. In some embodiments, the self-lubricating cannula is used to deliver insulin. In some embodiments, the self-lubricating cannula is a fluid conduit for a fluid medication. In some embodiments, the self-lubricating cannula is used to deliver insulin to a diabetic subject at a single site of infusion over a period of time (*e.g.* 1-2 days, 2-3 days, at least 6-7 days, at least 10 days).

### III. Evaluating Cannula Performance

Examples of evaluating cannula performance include monitoring the total daily dose (TDD) of insulin and determining a patient's comfort level during cannula insertion or during insulin infusion.

The TDD is the amount of insulin needed to maintain an individual's blood glucose at a desirable level. Foreign body response, tissue injury, and infusion site as a result of infusion device use (*i.e.* cannula implantation and insulin infusion) can diminish the effective delivery of insulin. When the body begins to react at the insertion site, the amount of insulin needed to maintain normal blood glucose levels of the patient will begin to increase. Eventually, no amount of insulin will reduce glucose levels, at which point the infusion set must be removed and a new site for infusion is needed. Hence, monitoring the TDD of insulin is useful in assessing the implanted cannula performance.

The subjective level of comfort and discomfort of cannula insertion can be assessed by providing test subjects with a questionnaire. Immediately following each cannula insertion, a comfort/discomfort Likert scale (very uncomfortable, uncomfortable, neutral, comfortable, and very comfortable) may be immediately presented to each study participant. The patient indicates which level is closest to that perceived during cannula penetration and the response is recorded. The word "pain" is used not in order to avoid influencing the perception of study participants. At the end of the test, the data is tabulated and analyzed in pairs ("conventional technique" x "emergent technique") with the aid of SPSS^{®} software.

Where the context permits, singular or plural terms may also include the plural or singular term, respectively. Although specific embodiments of, and examples for, the technology are described above for illustrative purposes, various equivalent modifications are possible within the scope of the technology, as those skilled in the relevant art will recognize.

As used herein, the terms "generally," "substantially," "about," and similar terms are used as terms of approximation and not as terms of degree, and are intended to account for the inherent variations in measured or calculated values that would be recognized by those of ordinary skill in the art. As used herein, the term "about" is used synonymously with the term "approximately". Illustratively, the use of the term "about" with regard to an amount indicates values slightly outside the cited values, e.g., plus or minus 0.1% to 10%, plus or minus 0.1% to 5%, or plus or minus 0.1% to 2%. In some embodiments, the term "about" indicates values plus or minus 10% of the cited values. In some embodiments, the term "about" indicates values plus or minus 5% of the cited values. In some embodiments, the term "about" indicates values plus or minus 2% of the cited values. In some embodiments, the term "about" indicates values plus or minus 1% of the cited values.

Moreover, unless the word "or" is expressly limited to mean only a single item exclusive from the other items in reference to a list of two or more items, then the use of "or" in such a list is to be interpreted as including (a) any single item in the list, (b) all of the items in the list, or (c) any combination of the items in the list. Additionally, the term "comprising" is used throughout to mean including at least the recited feature(s) such that any greater number of the same feature and/or additional types of other features are not precluded.

### IV. EXEMPLARY EMBODIMENTS

Some of the embodiments of this disclosure relate to Embodiment I, as follows:
Embodiment I-1. A self-lubricating cannula comprising a polyether block polyamide and an additive, wherein the self-lubricating cannula is configured for delivering insulin to an individual in need thereof and the self-lubricating cannula resists sticking to a heated mold.
Embodiment I-2. The self-lubricating cannula according to Embodiment I-1, wherein the additive is a siloxane.
Embodiment I-3. The self-lubricating cannula according to Embodiment I-1 or I-2, wherein the additive is a polysiloxane.
Embodiment I-4. The self-lubricating cannula according to Embodiment I-3, wherein the polysiloxane is a high molecular weight polysiloxane.
Embodiment I-5. The self-lubricating cannula according to any one of Embodiments I-1 to I-4, wherein the additive is polydimethylsiloxane.
Embodiment I-6. The self-lubricating cannula according to any one of Embodiments I-1 to I-5, wherein the additive is Multibase^{™} MB50-017.
Embodiment I-7. The self-lubricating cannula according to any one of Embodiments I-1 to I-6, wherein the self-lubricating cannula comprises: less than 10 wt%, less than 7 wt%, less than 5 wt%, less than 3 wt%, less than 2 wt %, less than 1.5 wt%, or less than 1 wt% of the additive, and at least 0.1 wt% of the additive.
Embodiment I-8. The self-lubricating cannula according to any one of Embodiments I-1 to I-6, wherein the self-lubricating cannula comprises: 0 .1 wt% to 10 wt%, 0.1 wt% to 7 wt%, 0.1 wt% to 5 wt%, 0.1 wt% to 3 wt%, 0.1 wt% to 2 wt%, 0.1 wt% to 1.5 wt%, 0.1 wt% to 1 wt%, 0.5 wt% to 10 wt%, 0.5 wt% to 7 wt%, 0.5 wt% to 5 wt%, 0.5 wt% to 3 wt%, 0.5 wt% to 2 wt%, 0.5 wt% to 1.5 wt%, 0.5 wt% to 1 wt%, 0.7 wt% to 10 wt%, 0.7 wt% to 7 wt%, 0.7 wt% to 5 wt%, 0.7 wt% to 3 wt%, 0.7 wt% to 2 wt%, 0.7 wt% to 1.5 wt%, 0.7 wt% to 1 wt%, 1 wt% to 10 wt%, 1 wt% to 7 wt%, 1 wt% to 5 wt%, 1 wt% to 3 wt%, 1 wt% to 2 wt%, or 1 wt% to 1.5 wt% of the additive.
Embodiment I-9. The self-lubricating cannula according to any one of Embodiments I-1 to I-8, wherein the self-lubricating cannula comprises about 10 wt%, about 9.5 wt%, about 9 wt%, about 8.5 wt%, about 8 wt%, about 7.5 wt%, about 7 wt%, about 6.5 wt%, about 6 wt%, about 5.5 wt%, about 5 wt%, about 4.5 wt%, about 4 wt%, about 3 .5 wt%, about 3 wt%, about 2.5 wt%, about 2 wt%, about 1.5 wt%, about 1 wt%, or about 0.5 wt % of the additive.
Embodiment I-10. The self-lubricating cannula according to any one of Embodiments I-1 to I-9, wherein the self-lubricating cannula further comprises thermoplastic polyurethane.
Embodiment I-11. The self-lubricating cannula according to any one of Embodiments I-1 to I-10, wherein the polyamide block polyether is PEBAX^{®}.
Embodiment I-12. The self-lubricating cannula according to any one of Embodiments I-1 to I-11, wherein: the polyamide block polyether is selected from the group consisting of PEBAX^{®} 3533, PEBAX^{®} 5533, and PEBAX^{®} 7233, or a combination thereof.
Embodiment I-13. The self-lubricating cannula according to any one of Embodiments I-1 to I-10, wherein: the polyamide block polyether has a physical property profile comprising two or more physical properties selected from the group consisting of shore hardness, density, melting point, humidity, absorption, water absorption, flexural modulus, and tensile modulus; and the physical property profile is equivalent to the physical property profile of an PEBAX^{®} polyether block polyamide described in Table 1.
Embodiment I-14. The self-lubricating cannula according to any one of Embodiments I-1 to I-10, wherein: the polyamide block polyether has a physical property profile comprising two or more physical properties selected from the group consisting of shore hardness, density, melting point, humidity, absorption, water absorption, flexural modulus, and tensile modulus; and the physical property profile is equivalent to the physical property profile of PEBAX^{®} 3533, PEBAX^{®} 5533, or PEBAX^{®} 7233, as described in Table 1.
Embodiment I-15. The self-lubricating cannula according to Embodiment I-13 or I-14, wherein: the polyamide block polyether has a physical property profile comprising three or more physical properties selected from the group consisting of shore hardness, density, melting point, humidity, absorption, water absorption, flexural modulus, and tensile modulus.
Embodiment I-16. The self-lubricating cannula according to Embodiment I-13 or I-14, wherein: the polyamide block polyether has a physical property profile comprising four or more physical properties selected from the group consisting of shore hardness, density, melting point, humidity, absorption, water absorption, flexural modulus, and tensile modulus.
Embodiment I-17. The self-lubricating cannula according to any one of Embodiments I-1 to I-16, wherein after contact of the self-lubricating cannula with a heated mold, the surface of the self-lubricating cannula having contact with the heated mold is smoother compared to the surface of an analogous additive-free cannula having contact with a heated mold.
Embodiment I-18. The self-lubricating cannula according to any one of Embodiments I-1 to I-17, wherein the distal end of the self-lubricating cannula comprises a tip configured to penetrate the skin of the individual in need thereof.
Embodiment I-19. The self-lubricating cannula according to any one of Embodiments I-1 to I-18, wherein the self-lubricating cannula does not contain polyfluoroalkyl substances (PFAS).
Embodiment I-20. The self-lubricating cannula according to any one of Embodiments I-1 to I-19, wherein the self-lubricating cannula further comprises a needle located within the self-lubricating cannula.
Embodiment I-21. The self-lubricating cannula according to any one of Embodiments I-1 to I-19, wherein the self-lubricating cannula further comprises a needle located on the exterior of the self-lubricating cannula.
Embodiment I-22. The self-lubricating cannula according to any one of Embodiments I-1 to I-21, wherein the individual in need thereof is diabetic.
Embodiment I-23. The self-lubricating cannula according to any one of Embodiments I-1 to I-22, wherein the self-lubricating cannula is configured for subcutaneous insertion into a tissue of a diabetic patient.
Embodiment I-24. The self-lubricating cannula according to Embodiment I-23, wherein the cannula is configured to be implanted for at least 2 days at a single site on the diabetic patient before removal.
Embodiment I-25. The self-lubricating cannula according to any one of Embodiments I-1 to I-24, wherein the cannula has a dimensional profile comprising two or more, three or more, four or more, five or more, six or more or seven or more dimensions within the dimensional ranges in Table 3.
Embodiment I-26. The self-lubricating cannula according to any one of Embodiments I-1 to I-25, wherein the cannula has dimensions that fall within all the dimensional ranges provided in Table 3.
Embodiment I-27. A method of making a self-lubricating cannula, comprising the steps of: combining a polyether block polyamide and an additive to form a mixture, extruding the mixture to form a cannula, cutting the cannula to a desired length, and treating the distal end of the cannula with a heated mold to form a tip; wherein the self-lubricating cannula is for use in delivering insulin to an individual in need thereof, and the self-lubricating cannula resists sticking to a heated mold.
Embodiment I-28. The method according to Embodiment I-27, wherein the heated mold is optionally lubricated prior to treating the distal end of the cannula with a heated mold to form a tip.
Embodiment I-29. The method according to Embodiment I-27 or I-28, wherein the additive is a siloxane.
Embodiment I-30. The method according to any one of Embodiments I-27 to I-29, wherein the additive is a polysiloxane.
Embodiment I-31. The method according to Embodiment I-30, wherein the polysiloxane is a high molecular weight polysiloxane.
Embodiment I-32. The method according to any one of Embodiments I-27 to I-31, wherein the additive is polydimethylsiloxane.
Embodiment I-33. The method according to any one of Embodiments I-27 to I-31, wherein the additive is Multibase^{™} MB50-017.
Embodiment I-34. The method according to any one of Embodiments I-27 to I-33, wherein the self-lubricating cannula comprises: less than than 10 wt%, less than 7 wt%, less than 5 wt%, less than 3 wt%, less than 2 wt %, less than 1.5 wt%, or less than 1 wt% of the additive, and at least 0.1 wt% of the additive.
Embodiment I-35. The method according to any one of Embodiments I-27 to I-33, wherein the self-lubricating cannula comprises: 0 .1 wt% to 10 wt%, 0.1 wt% to 7 wt%, 0.1 wt% to 5 wt%, 0.1 wt% to 3 wt%, 0.1 wt% to 2 wt%, 0.1 wt% to 1.5 wt%, 0.1 wt% to 1 wt%, 0.5 wt% to 10 wt%, 0.5 wt% to 7 wt%, 0.5 wt% to 5 wt%, 0.5 wt% to 3 wt%, 0.5 wt% to 2 wt%, 0.5 wt% to 1.5 wt%, 0.5 wt% to 1 wt%, 0.7 wt% to 10 wt%, 0.7 wt% to 7 wt%, 0.7 wt% to 5 wt%, 0.7 wt% to 3 wt%, 0.7 wt% to 2 wt%, 0.7 wt% to 1.5 wt%, 0.7 wt% to 1 wt%, 1 wt% to 10 wt%, 1 wt% to 7 wt%, 1 wt% to 5 wt%, 1 wt% to 3 wt%, 1 wt% to 2 wt%, or 1 wt% to 1.5 wt% of the additive.
Embodiment I-36. The method according to any one of Embodiments I-27 to I-33, wherein the self-lubricating cannula comprises about 10 wt%, about 9.5 wt%, about 9 wt%, about 8.5 wt%, about 8 wt%, about 7.5 wt%, about 7 wt%, about 6.5 wt%, about 6 wt%, about 5.5 wt%, about 5 wt%, about 4.5 wt%, about 4 wt%, about 3 .5 wt%, about 3 wt%, about 2.5 wt%, about 2 wt%, about 1.5 wt%, about 1 wt%, or about 0.5 wt % of the additive.
Embodiment I-37. The method according to any one of Embodiments I-27 to I-36, wherein the additive is dispersed in thermoplastic polyurethane.
Embodiment I-38. The method according to any one of Embodiments I-27 to I-37, wherein the polyamide block polyether is PEBAX^{®}.
Embodiment I-39. The method according to any one of Embodiments I-27 to I-38, wherein the polyamide block polyether is selected from the group consisting of PEBAX^{®} 3533, PEBAX^{®} 5533, and PEBAX^{®} 7233, or a combination thereof.
Embodiment I-40. The method according to any one of Embodiments I-27 to I-39, wherein: the polyamide block polyether has a physical property profile comprising two or more physical properties selected from the group consisting of shore hardness, density, melting point, humidity, absorption, water absorption, flexural modulus, and tensile modulus; and the physical property profile is equivalent to the physical property profile of an PEBAX^{®} polyether block polyamide described in Table 1.
Embodiment I-41. The method according to any one of Embodiments I-27 to I-40, wherein: the polyamide block polyether has a physical property profile comprising two or more physical properties selected from the group consisting of shore hardness, density, melting point, humidity, absorption, water absorption, flexural modulus, and tensile modulus; and the physical property profile is equivalent to the physical property profile of PEBAX^{®} 3533, PEBAX^{®} 5533, or PEBAX^{®} 7233, as described in Table 1.
Embodiment I-42. The method according to Embodiment I-40 or I-41, wherein: the polyamide block polyether has a physical property profile comprising three or more physical properties selected from the group consisting of shore hardness, density, melting point, humidity, absorption, water absorption, flexural modulus, and tensile modulus.
Embodiment I-43. The method according to Embodiment I-40 or I-41, wherein: the polyamide block polyether has a physical property profile comprising four or more physical properties selected from the group consisting of shore hardness, density, melting point, humidity, absorption, water absorption, flexural modulus, and tensile modulus.
Embodiment I-44. The method according to any one of Embodiments I-27 to I-43, wherein the surface of the self-lubricating cannula having contact with the heated mold is smoother compared to the surface of an analogous additive-free cannula having contact with a heated mold.
Embodiment I-45. The method according to any one of Embodiments I-27 to I-44, wherein the distal end of the self-lubricating cannula comprises a tip configured to penetrate the skin of the individual in need thereof.
Embodiment I-46. The method according to any one of Embodiments I-27 to I-45, wherein the self-lubricating cannula does not contain polyfluoroalkyl substances (PFAS).
Embodiment I-47. The method according to any one of Embodiments I-27 to I-46, wherein the self-lubricating cannula further comprises a needle located within the self-lubricating cannula.
Embodiment I-48. The method according to any one of Embodiments I-27 to I-46, wherein the self-lubricating cannula further comprises a needle located on the exterior of the self-lubricating cannula.
Embodiment I-49. The method according to any one of Embodiments I-27 to I-48, wherein the individual in need thereof is diabetic.
Embodiment I-50. The method according to any one of Embodiments I-27 to I-49, wherein the self-lubricating cannula is configured for subcutaneous insertion into a tissue of a diabetic human.
Embodiment I-51. The method according to Embodiment I-50, wherein the self-lubricating cannula is configured to be implanted for at least 3 days at a single site on the diabetic human before removal.
Embodiment I-52. A self-lubricating cannula formed by the method of any one of claims 27-51.
Embodiment I-53. A self-lubricating cannula for use in delivering insulin to an individual in need thereof according to any one of Embodiments I-1 to I-26 or I-52.
Embodiment I-54. A method of administering insulin to an individual in need thereof, comprising: providing a self-lubricating cannula according to any one of Embodiments I-1 to I-26, I-52, or I-53; inserting the self-lubricating cannula into the individual; and delivering insulin via the self-lubricating cannula into the individual in need thereof.
Embodiment I-55. The method according to Embodiment I-54, wherein the self-lubricating cannula is inserted in subcutaneous tissue on the abdomen, thighs, hips, upper arms, lower back, or buttocks of the individual in need thereof.
Embodiment I-56. The method according to Embodiment I-53 or I-54, wherein the self-lubricating cannula does not bunch or kink during insertion into the individual in need thereof.
Embodiment I-57. The method according to any one of Embodiments I-53 to I-56, wherein the self-lubricating cannula is implanted in the individual in need thereof for at least 3, at least 4, at least 5, at least 6, at least 7, at least 8, at least 9, or at least 10 continuous days before the cannula is replaced or removed.
Embodiment I-58. The method according to any one of Embodiments I-53 to I-57, wherein the Total Daily Dose (TDD) of insulin delivered to an individual in need thereof does not increase by more than at least 25% than the average TDD over a period of up to 10 continuous days.
Embodiment I-59. The method according to any one of Embodiments I-53 to I-58, wherein the cannula is in fluid connection with an insulin infusion device.
Embodiment I-60. An infusion device comprising: a housing configured to be positioned at the skin of a patient at an infusion site; a reservoir configured to store a fluid medication, the reservoir configured to be received by the housing; and a cannula configured for subcutaneous insertion into a tissue of the patient at the infusion site, wherein the cannula is configured to be self-lubricating and comprises a polyether block polyamide and an additive.
Embodiment I-61. The infusion device according to Embodiment I-60, wherein the cannula is a self-lubricating cannula according to any one of Embodiments I-1 to I-26, I-52, or I-53.

### V. EXAMPLES

The following specific examples are to be construed as merely illustrative, and not limitative of the remainder of the disclosure in any way whatsoever.

### Example 1: General Process For Preparing Self-lubricating Cannulas

This example describes a general process for preparing self-lubricating cannulas described herein.

Polyether block amide (*e.g.* PEBAX^{®} resin) and siloxane additive (*e.g.* 3 wt% of Dow Corning MB50-017 Masterbatch) were combined and blended to incorporate the siloxane throughout the polymer material. The raw material containing 1.5 wt% siloxane was added to a feed hopper and extruded on conventional screw extrusion equipment. Specific processing parameters related to the particular resin(s) were obtained from the supplier. The extruded catheter was cut to length and flared onto a mandrel mounted bushing by interference fitting. The distal end of the catheter was then tip-formed using a heated and lubricated mold. The process required pre-heating a tip-forming mold to a predetermined temperature in order to reflow the resin and force the tip of the catheter to conform to the shape of the mold. A silicone/siloxane lubricant was applied to the heated mold surfaces just prior to tip-forming. The distal end of the catheter is forced and pressed into the heated mold. A mandrel was used to hold, guide, and center the catheter with the heated mold during tip-forming. The distal end was held in the mold for a predetermined dwell time to enable the distal end of the catheter in contact with the mold to reflow and conform to the dimensions of the mold. After the predetermined dwell time was completed, the tip-formed cannula was retracted from the mold.

The type of PEBAX^{®} resin used to make the catheter determines in part the molding conditions, such as mold temperature. For instance, Table 2 contains molding conditions for various Pebax^{®}.

**Table 2.**

| **Grades** | | **7233** | **7033** | **6333** | **5533** | **4533** | **4033** | **3533** | **2533** |
|---|---|---|---|---|---|---|---|---|---|
| Melt Temperature (°C) | | | | | | | | | |
| | Minimum | 230 | 230 | 230 | 200 | 200 | 200 | 180 | 180 |
| | Recommended | 260 | 260 | 260 | 240 | 240 | 240 | 210 | 210 |
| | Maximum | 290 | 290 | 290 | 270 | 270 | 270 | 240 | 240 |
| Mold Temperature (°C) | | | | | | | | | |
| | Recommended | 25 - 60 | 25 - 60 | 25 - 60 | 25 - 60 | 10 - 30 | 10 - 30 | 10 - 30 | 10 - 30 |
| Drying Conditions* | | | | | | | | | |
| | Temperature (°C) | 70 - 80 | 70 - 80 | 65 - 75 | 65 - 75 | 60 - 70 | 60 - 70 | 55 - 65 | 55 - 65 |
| | Duration (Hr) | 5 - 7 | 5 - 7 | 4 - 8 | 4 - 8 | 4 - 8 | 4 - 8 | 4 - 8 | 4 - 8 |

| | | | | | | | | | |
|---|---|---|---|---|---|---|---|---|---|
| *: Dessicant dryer (dew point ≤ -25 °C) | | | | | | | | | |

### Example 2: Comparison of Polyether Block Polyamide With and Without Siloxane

This example compares a self-lubricating cannula to a cannula formed without an additive. In particular, visual inspection of the tipped surface and form reveals different results from the tipping process.

Cannulas comprising polyether block polyamide (PEBAX^{®} 5533) and 1.5 wt% of siloxane polymer (3% of Dow Corning MB50-017 Masterbatch, composed of ultra-high molecular weight polysiloxane dispersed in thermoplastic polyurethane, wherein the siloxane content is 50%) were prepared as described in Example 1.

An analogous cannula was also prepared under the same conditions but without the addition of the additive. It was noted that the additive-free cannula tip adhered to the mold and could not be retracted without damaging the tip even when siloxane lubricants were applied to the heated mold prior to tip-forming. The cannula tips were examined visually using an optical microscope.

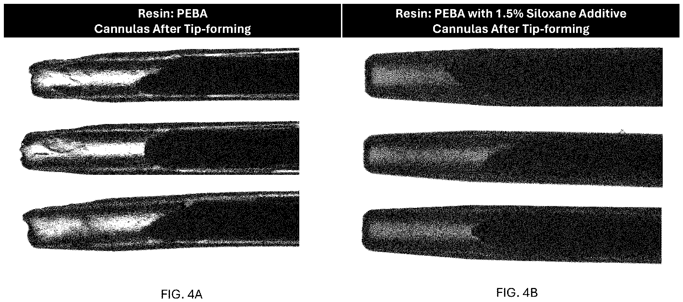

FIGS. 4 show photos of polyether block polyamide cannula tips under magnification. The tip of the cannula without additive (FIG. 4A) has a rough and irregular surface. The end of the tip is ragged. In contrast, the self-lubricating cannula comprising 1.5% polysiloxane shown in FIG. 4B has a comparably smoother surface and evenly rounded tip ends.

Polyether block polyamide is known to be an inherently sticky material. As observed in this example, when used alone in the formation of a cannula, polyether block polyamide sticks to the heated mold during the tip-forming process, resulting in poorly formed tips - the surfaces and edges are rough, irregular, and inconsistent. Cannulas formed from polyether block polyamide alone adhered to the tip-forming mold, even when excessive lubricant was added to the mold.

The incorporation of 1.5 wt% siloxane to the cannula material improved the manufacturability and cannula quality as observed in photo of FIG. 4B. This example demonstrates the feasibility of fabricating cannulas using polyether block polyamides and a small amount of siloxane additive under existing manufacturing processes. With siloxane, cannula has improved reflow characteristics while also not adhering to the molding surfaces during tip-forming.

### Example 3: Exemplary Cannula Specification

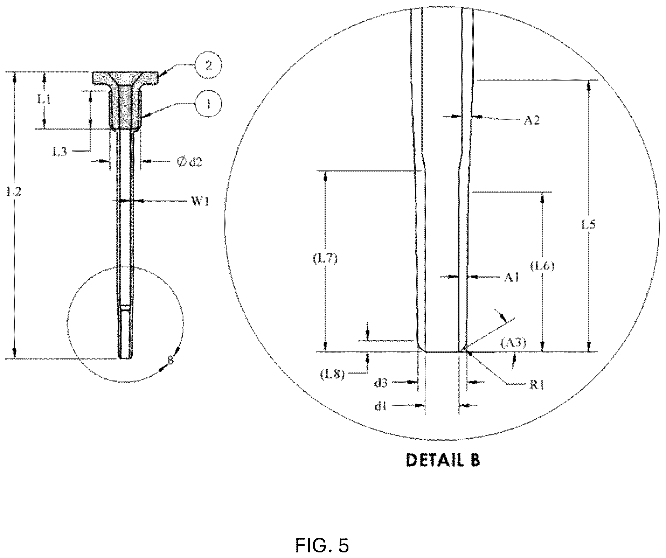

This example provides a dimensional description of an exemplary cannula disclosed herein. Table 3 provides the dimensions of various parameters of the cannula and bushing/needle guide shown in FIG. 5. Feature 1 shows the proximal end of the cannula flared onto to a needle guide/bushing labeled as Feature 2 and is the fluid inlet of the cannula.

**Table 3.**

| **Dimension** | **Description** | **Dimensional Ranges** |
|---|---|---|
| L1 | Overall Bushing Length On Bushing | 1.0 to 6.0mm |
| L2 | Overall Subassembly Length | 7.0 to 20.0mm |
| L3 | Flare Length | 0.5 to 5.0 mm |
| W1 | Catheter Tubing Wall Thickness | 0.05 to 0.5mm |
| d1 | Mandrel Diameter at Tip / Catheter Tip Inner Diameter | 0.1 to 1.0 mm |
| d3 | Catheter Tip Outer Diameter | 0.1 to 2.0 mm |
| R1 | Tip Forming Die Dimension: Catheter Tip Radius | 0.05 to 0.5 mm |
| L5 | Tip Forming Die Dimension: Overall Lead-In Taper Length | 0.5 to 5.0 mm |
| A1 | Primary Tip Angle | 1.0 to 5.0 deg |

In some embodiments, the disclosure provides a self-lubricating cannula having a dimensional profile comprising two or more, three or more, four or more, five or more, six or more or seven or more dimensions within the dimensional ranges in Table 3. In some embodiments, the self-lubricating cannula has dimensions that fall within all the dimensional ranges provided in Table 3.

### Example 4: Preclinical testing of Cannulas in a Diabetic Porcine Model

### Example 3: Preclinical Testing of Cannulas in a Diabetic Porcine Model

The performance of a prototype self-lubricating cannula used for insulin infusion may be evaluated in a diabetic porcine model over a period of time *(e.g.* 10 days). Commercially available infusion devices can be modified to accommodate the test cannulas. The performance of prototype cannulas can be tested against a commercially available cannula standard, for instance the Medtronic Extended^{™} Infusion Set (EIS).

The animal subjects are fasted for over 12 hours prior to testing. On Day 0, the pigs are fed a standardized diet at prescribed times. A cannula is placed subcutaneously into abdominal tissue while the subject is under general anesthesia. A glucose sensor is also implanted at least 5 cm from the cannula. In addition, blood glucose levels are measured 5 times daily at prescribed times (*i.e.* 07:30, 09:30, 11:30, 14:30, and 16:30). The infusion device is used to deliver Novolog insulin into the subjects.

The basal rate of delivery is established for Day 0, which represents the basal rate of delivery needed to maintain blood glucose concentrations at a desired range (*e.g.* 100 - 400 mg/dL). The infusion device delivers bolus amounts of insulin as needed, for instance after meals when the blood glucose concentration increases beyond the desired range. The infusion set is considered failed when the blood glucose concentration does not respond to an insulin dose change, for instance when administering increasing amounts of insulin fail to reduce or control blood glucose concentrations.

The amount of insulin administered over a 24-hour period, Total Daily Dose (TDD), is calculated over the test period or until the infusion set fails. The survival rate of the animals can be compared as well as the variance in TDD of insulin over the test duration. An increase in the TDD of insulin over time may indicate deterioration of the infusion site. If TDD of insulin does not vary much, it suggests successful delivery of insulin and the maintenance of blood glucose concentrations at the desired levels. In addition, the performance of the test cannula can be compared to the performance of the commercial standard.

### Conclusion

While embodiments of the present invention have been shown and described herein, those skilled in the art will understand that such embodiments are provided by way of example only. Numerous variations, changes, and substitutions will now occur to those skilled in the art without departing from the invention. It should be understood that various alternatives to the embodiments of the invention described herein may be employed in practicing the invention. For example, while steps are presented in a given order, alternative embodiments may perform steps in a different order. The various embodiments described herein may also be combined to provide further embodiments. It is intended that the following claims define the scope of the invention and that methods and structures within the scope of these claims and their equivalents be covered thereby.

It will also be appreciated that specific embodiments have been described herein for purposes of illustration, but that various modifications may be made without deviating from the technology. Further, while advantages associated with certain embodiments of the technology have been described in the context of those embodiments, other embodiments may also exhibit such advantages, and not all embodiments need necessarily exhibit such advantages to fall within the scope of the technology. Accordingly, the disclosure and associated technology can encompass other embodiments not expressly shown or described herein.

The following examples are illustrative of the techniques described herein.

Example 1: A self-lubricating cannula comprising a polyether block polyamide and an additive, wherein the self-lubricating cannula is configured for delivering insulin to an individual in need thereof and the self-lubricating cannula resists sticking to a heated mold.

Example 2: The self-lubricating cannula according to example 1, wherein the additive is a polysiloxane.

Example 3: The self-lubricating cannula according to example 2, wherein the polysiloxane is a high molecular weight polysiloxane.

Example 4: The self-lubricating cannula according to example 1, wherein the self-lubricating cannula comprises:
0 .1 wt% to 10 wt%, 0.1 wt% to 7 wt%, 0.1 wt% to 5 wt%, 0.1 wt% to 3 wt%, 0.1 wt% to 2 wt%, 0.1 wt% to 1.5 wt%, 0.1 wt% to 1 wt%,
0.5 wt% to 10 wt%, 0.5 wt% to 7 wt%, 0.5 wt% to 5 wt%, 0.5 wt% to 3 wt%, 0.5 wt% to 2 wt%, 0.5 wt% to 1.5 wt%, 0.5 wt% to 1 wt%,
0.7 wt% to 10 wt%, 0.7 wt% to 7 wt%, 0.7 wt% to 5 wt%, 0.7 wt% to 3 wt%, 0.7 wt% to 2 wt%, 0.7 wt% to 1.5 wt%, 0.7 wt% to 1 wt%,
1 wt% to 10 wt%, 1 wt% to 7 wt%, 1 wt% to 5 wt%, 1 wt% to 3 wt%, 1 wt% to 2 wt%, or 1 wt% to 1.5 wt% of the additive.

Example 5: The self-lubricating cannula according to example 1, wherein the self-lubricating cannula further comprises thermoplastic polyurethane.

Example 6: The self-lubricating cannula according to example 1, wherein the polyamide block polyether is PEBAX^{™}.

Example 7: The self-lubricating cannula according to example 1, wherein:
the polyamide block polyether has a physical property profile comprising two or more physical properties selected from the group consisting of shore hardness, density, melting point, humidity, absorption, water absorption, flexural modulus, and tensile modulus; and
the physical property profile is equivalent to the physical property profile of an PEBAX^{®} polyether block polyamide described in Table 1.

Example 8: The self-lubricating cannula according to example 1, wherein the self-lubricating cannula does not contain polyfluoroalkyl substances (PFAS).

Example 9: The self-lubricating cannula according to example 1, wherein the self-lubricating cannula is configured for subcutaneous insertion into a tissue of a diabetic patient.

Example 10: A method of making a self-lubricating cannula, comprising the steps of:
combining a polyether block polyamide and an additive to form a mixture,
extruding the mixture to form a cannula,
cutting the cannula to a desired length, and
treating the distal end of the cannula with a heated mold to form a tip;
wherein the self-lubricating cannula is for use in delivering insulin to an individual in need thereof, and the self-lubricating cannula resists sticking to a heated mold.

Example 11: The method according to example 10, wherein the additive is a polysiloxane.

Example 12: The method according to example 10, wherein the self-lubricating cannula comprises:
0 .1 wt% to 10 wt%, 0.1 wt% to 7 wt%, 0.1 wt% to 5 wt%, 0.1 wt% to 3 wt%, 0.1 wt% to 2 wt%, 0.1 wt% to 1.5 wt%, 0.1 wt% to 1 wt%,
0.5 wt% to 10 wt%, 0.5 wt% to 7 wt%, 0.5 wt% to 5 wt%, 0.5 wt% to 3 wt%, 0.5 wt% to 2 wt%, 0.5 wt% to 1.5 wt%, 0.5 wt% to 1 wt%,
0.7 wt% to 10 wt%, 0.7 wt% to 7 wt%, 0.7 wt% to 5 wt%, 0.7 wt% to 3 wt%, 0.7 wt% to 2 wt%, 0.7 wt% to 1.5 wt%, 0.7 wt% to 1 wt%,
1 wt% to 10 wt%, 1 wt% to 7 wt%, 1 wt% to 5 wt%, 1 wt% to 3 wt%, 1 wt% to 2 wt%, or 1 wt% to 1.5 wt% of the additive.

Example 13: The method according to example 10, wherein the additive is dispersed in thermoplastic polyurethane.

Example 14: The method according to example 10, wherein:
the polyamide block polyether has a physical property profile comprising two or more physical properties selected from the group consisting of shore hardness, density, melting point, humidity, absorption, water absorption, flexural modulus, and tensile modulus; and
the physical property profile is equivalent to the physical property profile of an PEBAX^{®} polyether block polyamide described in Table 1.

Example 15: The method according to example 10, wherein the self-lubricating cannula does not contain polyfluoroalkyl substances (PFAS).

Example 16: A method of administering insulin to an individual in need thereof, comprising:
providing a self-lubricating cannula according to example 1;
inserting the self-lubricating cannula into the individual; and
delivering insulin via the self-lubricating cannula into the individual in need thereof.

Example 17: The method according to example 16, wherein the self-lubricating cannula is implanted in the individual in need thereof for at least 3, at least 4, at least 5, at least 6, at least 7, at least 8, at least 9, or at least 10 continuous days before the cannula is replaced or removed.

Example 18: The method according to example 16, wherein the Total Daily Dose (TDD) of insulin delivered to an individual in need thereof does not increase by more than at least 25% than the average TDD over a period of up to 10 continuous days.

Example 19: An infusion device comprising:
a housing configured to be positioned at the skin of a patient at an infusion site;
a reservoir configured to store a fluid medication, the reservoir configured to be received by the housing; and
a cannula configured for subcutaneous insertion into a tissue of the patient at the infusion site, wherein the cannula is configured to be self-lubricating and comprises a polyether block polyamide and an additive.

Example 20: The infusion device according to example 19, wherein the cannula is a self-lubricating cannula according to example 1.

## Claims

1. A self-lubricating cannula comprising a polyether block polyamide and an additive, wherein the self-lubricating cannula is configured for delivering insulin to an individual in need thereof and the self-lubricating cannula resists sticking to a heated mold.

2. The self-lubricating cannula according to claim 1, wherein the additive is a polysiloxane.

3. The self-lubricating cannula according to claim 2, wherein the polysiloxane is a high molecular weight polysiloxane.

4. The self-lubricating cannula according to any one of claims 1-3, wherein the self-lubricating cannula comprises:
0 .1 wt% to 10 wt%, 0.1 wt% to 7 wt%, 0.1 wt% to 5 wt%, 0.1 wt% to 3 wt%, 0.1 wt% to 2 wt%, 0.1 wt% to 1.5 wt%, 0.1 wt% to 1 wt%,
0.5 wt% to 10 wt%, 0.5 wt% to 7 wt%, 0.5 wt% to 5 wt%, 0.5 wt% to 3 wt%, 0.5 wt% to 2 wt%, 0.5 wt% to 1.5 wt%, 0.5 wt% to 1 wt%,
0.7 wt% to 10 wt%, 0.7 wt% to 7 wt%, 0.7 wt% to 5 wt%, 0.7 wt% to 3 wt%, 0.7 wt% to 2 wt%, 0.7 wt% to 1.5 wt%, 0.7 wt% to 1 wt%,
1 wt% to 10 wt%, 1 wt% to 7 wt%, 1 wt% to 5 wt%, 1 wt% to 3 wt%, 1 wt% to 2 wt%, or 1 wt% to 1.5 wt% of the additive.

5. The self-lubricating cannula according to any one of claims 1-4, wherein the self-lubricating cannula further comprises thermoplastic polyurethane.

6. The self-lubricating cannula according to any one of claims 1-5, wherein:
the polyamide block polyether has a physical property profile comprising two or more physical properties selected from the group consisting of shore hardness, density, melting point, humidity, absorption, water absorption, flexural modulus, and tensile modulus; and
the physical property profile is equivalent to the physical property profile of an PEBAX^{®} polyether block polyamide described in Table 1.

7. The self-lubricating cannula according to any one of claims 1-6, wherein the self-lubricating cannula does not contain polyfluoroalkyl substances (PFAS).

8. The self-lubricating cannula according to any one of claims 1-7, wherein the self-lubricating cannula is configured for subcutaneous insertion into a tissue of a diabetic patient.

9. A method of making a self-lubricating cannula, comprising the steps of:
combining a polyether block polyamide and an additive to form a mixture,
extruding the mixture to form a cannula,
cutting the cannula to a desired length, and
treating the distal end of the cannula with a heated mold to form a tip;
wherein the self-lubricating cannula is for use in delivering insulin to an individual in need thereof, and the self-lubricating cannula resists sticking to a heated mold.

10. The method according to claim 9, wherein the additive is a polysiloxane.

11. The method according to any one of claims 9-10, wherein the self-lubricating cannula comprises:
0 .1 wt% to 10 wt%, 0.1 wt% to 7 wt%, 0.1 wt% to 5 wt%, 0.1 wt% to 3 wt%, 0.1 wt% to 2 wt%, 0.1 wt% to 1.5 wt%, 0.1 wt% to 1 wt%,
0.5 wt% to 10 wt%, 0.5 wt% to 7 wt%, 0.5 wt% to 5 wt%, 0.5 wt% to 3 wt%, 0.5 wt% to 2 wt%, 0.5 wt% to 1.5 wt%, 0.5 wt% to 1 wt%,
0.7 wt% to 10 wt%, 0.7 wt% to 7 wt%, 0.7 wt% to 5 wt%, 0.7 wt% to 3 wt%, 0.7 wt% to 2 wt%, 0.7 wt% to 1.5 wt%, 0.7 wt% to 1 wt%,
1 wt% to 10 wt%, 1 wt% to 7 wt%, 1 wt% to 5 wt%, 1 wt% to 3 wt%, 1 wt% to 2 wt%, or 1 wt% to 1.5 wt% of the additive.

12. The method according to any one of claims 9-11, wherein:
the polyamide block polyether has a physical property profile comprising two or more physical properties selected from the group consisting of shore hardness, density, melting point, humidity, absorption, water absorption, flexural modulus, and tensile modulus; and
the physical property profile is equivalent to the physical property profile of an PEBAX^{®} polyether block polyamide described in Table 1.

13. The method according to any one of claims 9-12, wherein the self-lubricating cannula does not contain polyfluoroalkyl substances (PFAS).

14. A method of administering insulin to an individual in need thereof, comprising:
providing a self-lubricating cannula according to any one of claims 1-8;
inserting the self-lubricating cannula into the individual; and
delivering insulin via the self-lubricating cannula into the individual in need thereof.

15. An infusion device comprising:
a housing configured to be positioned at the skin of a patient at an infusion site;
a reservoir configured to store a fluid medication, the reservoir configured to be received by the housing; and
a cannula configured for subcutaneous insertion into a tissue of the patient at the infusion site, wherein the cannula is configured to be self-lubricating and comprises a polyether block polyamide and an additive.
